# EUROPÄISCHE PATENTSCHRIFT

(11) **EP 1 434 864 B1**
(45) Veröffentlichungstag und Bekanntmachung des Hinweises auf die Patenterteilung: **08.06.2011**
(21) Anmeldenummer: 02779144.1
(22) Anmeldetag: 08.10.2002
(51) Int. Cl.: C12N 15/10

(54) **VERFAHREN ZUR UNSPEZIFISCHEN ANREICHERUNG VON BAKTERIENZELLEN**
METHOD FOR UNSPECIFIC ENRICHMENT OF BACTERIA CELLS
PROCEDE D'ENRICHISSEMENT NON SPECIFIQUE DE CELLULES DE BACTERIES

(30) Priorität: 09.10.2001 DE 10149803; 04.07.2002 DE 10230147
(43) Veröffentlichungstag der Anmeldung: 07.07.2004
(73) Patentinhaber: Hyglos Invest GmbH, 82347 Bernried (DE)
(72) Erfinder: DILLER, Sabine, 92421 Schwandorf (DE); GRASSL, Renate, 93049 Regensburg (DE); MILLER, Stefan, 93053 Regensburg (DE); ROBL, Ingrid, 93055 Regensburg (DE); SCHÜTZ, Michael, 93138 Kareth - Lappersdorf (DE); ZANDER, Thomas, 93138 Lappersdorf (DE)
(74) Vertreter: Dehmel, Albrecht
(86) Internationale Anmeldenummer: PCT/DE2002/003790
(87) Internationale Veröffentlichungsnummer: WO 2003/033698

(56) Entgegenhaltungen:
- WO-A-00/29562
- WO-A-98/51693
- GOLDBERG S ET AL: "MECHANISM OF ENHANCEMENT OF MICROBIAL CELL HYDROPHOBICITY BY CATIONIC POLYMERS" JOURNAL OF BACTERIOLOGY, Bd. 172, Nr. 10, 1990, Seiten 5650-5654, XP009005131 ISSN: 0021-9193 in der Anmeldung erwähnt

## Beschreibung

Die vorliegende Erfindung betrifft ein Verfahren zur unspezifischen Anreicherung von Bakterienzellen mittels kationischer oder anionischer Polymere und magnetischer Träger.

Ausgangspunkt fast jeder Weiterverarbeitung, Analyse oder Isolierung von Zellbestandteilen ist die Anreicherung der Zellen, die "Zellernte". Diese wird üblicherweise mittels Zentrifugation durchgeführt. Dieser Zentrifugationsschritt stellt das Hauptproblem bei der vollständigen Automatisierung von Verfahren, wie der Plasmidaufreinigung dar, da neben dem hohen technischen Aufwand zur Integration einer Zentrifuge in einem entsprechenden Verarbeitungsroboter eine extrem hohe Präzision bei Start- und Stop-Position des Zentrifugationsvorganges benötigt wird. Automatische Verfahren der Weiterverarbeitung, Analyse oder Isolierung von Zellbestandteilen beginnen daher in der Regel mit bereits ausserhalb des Verarbeitungsroboters angereicherten, abzentrifügierten oder sedimentierten Zellen. Jedoch ist zum Beispiel für eine schnelle Analyse von vollständigen Genomen, Proteomen, aber auch der raschen Struktur- und Funktionsaufklärung in Hochdurchsatzverfahren eine möglichst vollständige Automatisierung der dabei relevanten Verfahren essentiell. Dabei ist die Automatisierung von Teilschritten z.B. in der Genomanalyse bereits weit fortgeschritten: Sowohl die Bakterienanzucht, als auch die Plasmidisolierung können automatisch durchgeführt werden. Eine einfache, kostengünstige, vollständige Automatisierung des Verfahrens einschließlich der Zellernte ist bisher jedoch nicht durchführbar.

Das Standardverfahren der Zellernte ist die Abzentrifugation der Bakterienkulturen. Dazu ist gerade bei Verfahren, die für höheren Durchsatz ausgelegt werden sollen, eine Mikrotiterplattenzentrifuge nötig.

Ein alternatives Verfahren bedient sich der Anreicherung der kultivierten Zellen über Filtermembranen siehe z.B. WO01/51206, 3M Innovative Properties Co (US) und US5, 464, 541, Diagen Inst. f. Molekularbiologie, jedoch ist das Verfahren bei hochangereicherten Zellsuspensionen und der damit einhergehenden hohen Viskosität der Lösungen sehr störanfällig bezüglich Verstopfungen. Ähnliches gilt für Ionenaustauscher-ähnliche Bindung an eine poröse Matrix kombiniert mit Filtrations- oder Vakuumtechniken (WO92/07863, Qiagen). Dies kann gerade automatische Verfahren vor große Probleme stellen.

Eine Anreicherung von Mikroorganismen mittels Magnetpartikel-basierten Verfahren ist prinzipiell zur Automatisierung geeignet. Bislang wurden drei Anreicherungsverfahren für Bakterien beschrieben, die allerdings deutliche Nachteile in punkto Stammspezifität und Herstellungskosten aufweisen.

EP 1 113676 A2, Chemagen AG und WO 01/53525 A2, Genpoint AS beschreiben ein Verfahren zur Anreicherung von Mikroorganismen an einer Festphase über einen nicht-spezifischen Liganden, der an dieser Festphase kovalent oder nicht-kovalent immobilisiert ist. Festphasen im Sinne dieses Verfahrens sind Polyinylalkoholbeads die magnetisch oder nicht magnetisch sein können. Als Liganden werden im Sinne dieses Verfahrens Moleküle betrachtet, die den Mikroorganismen als Nährstoffe dienen können. Der Nachteil dieses Verfahrens besteht darin, dass man bestimmte Liganden an einem Träger immobilisieren muss, was zu einer deutlichen Erhöhung der Herstellungskosten führt. Ein weiterer Nachteil besteht darin, dass für verschiedene Stämme unter Umständen verschiedene Liganden immobilisiert werden müssen, die Magnetbeads und somit das Verfahren nicht universell einsetzbar sind.

In einer weiteren Anmeldung, nämlich WO98/51696, Genpoint, werden zur Anreicherung von Bakterien an Magnetpartikel Gemische aus Salzen, Alkoholen und Polyethylenglykol oder mit Polyethylenglykol vergleichbaren Polymere beschrieben. Das in WO98/51693 beschriebene Verfahren benötigt 5-20 Minuten und Polymerzugaben von 2-50 % (w/v). Diese hohen Konzentrationen von Salzen oder Polymeren bereiten Probleme in der Weiterverarbeitung der Bakterien, da z.B. Polyethylenglykol bei 30% sehr zähflüssig ist, oder hohe Salzkonzentrationen bei SDS-Polyacrylamidgelelektrophorese störend wirken.

WO00/29562, Merck Patent GmbH, beschreibt ein Verfahren zur Isolierung von Plasmid-DNA aus Mikroorganismen durch a) Ansäuern der Mikroorganismenkultur, Inkubation und Mischung mit den Festphasenkölpern (Silica-Magnetpartikeln) und b) anschliessende Plasmidaufreinigung. Dieses Verfahren ist jedoch nicht für alle E.coli-Stämme ohne z.T. drastische Ausbeuteverluste durchführbar. Ein weiterer Nachteil dieses Verfahrens liegt in der benötigten Beadmenge, da die Beadkosten den Schwerpunkt der Bakterienanreicherungskosten ausmachen und insbesondere Silica-Beads relativ teuer sind. Da Silica-Beads auch prinzipiell DNA binden können, können auch Ausbeuteverluste bei der Plasmidisolierung mit Silicabeads beobachtet werden.

WO91/12079, Amersham beschreibt ein Verfahren zur Präzipitation von Zellen an magnetischen Beads, die unspezifisch die Polymere (Zellen) binden. In diesem Verfahren ist die Reihenfolge der Zugabe der nötigen Agentien (Alkohol und Salz) und der magnetischen Beads zwingend einzuhalten. Ein Vormischen der Agentien und/oder Agentien und Beads ist nicht möglich und daher können keine Pipettierschritte eingespart werden, was bei einer Automatisierung für die optimale Ausnutzung eines Roboters nötig sein kann. Darüberhinaus sind die einsetzbaren Magnetpartikel auf Cellulose/Eisenoxid-Partikel beschränkt.

US 6,284,470 B1, Promega, beschreibt die Komplexbildung von intakten Zellen mit magnetischen Beads. Das Verfahren ist hinsichtlich der einsetzbaren Magnetpartikel beschränkt auf Silica-Partikel. Da dieses Verfahren aber vorschreibt, dass zu einem Volumen Zellsuspension ein Volumen Alkohol zugegeben werden muß, ist dieses Verfahren für die Zellernte in Deepwellplatten ohne deutliche Ausbeuteverluste kaum anwendbar. Darüberhinaus können hohe Konzentrationen Alkohol zu einer Präzipitation der Proteine führen.

Eine Reihe von Untersuchungen hatte gezeigt, dass kationische Polymere mit Bakterien interagieren können. Experimente hatten z.B. ergeben, dass kationische Polymere wie z.B. Chitosan (Chitosan ist deacetyliertes Chitin) sich gut zum Verkapseln oder "Eingiessen" lebender mikrobieller Zellen für Einzelzellexperimente eignen (siehe z. B. Methods Enzymol. 1987, Vol. 135, 259-268, oder US5,489,401). Chitosan findet auch Verwendung für die Verkapselung von aktiven Biomaterialien in Beadpolymeren (US4,647,536 und WO01/46266). Chitosan findet auch - gegebenenfalls auch in modifizierter Form - Verwendung als Beschichtung von Artikeln oder Testkits für Immunoassays (US5,208,166).

Veröffentlichungen zum Einfluß kationischer Polymere auf die Hydrophobizität von mikrobiellen Zellwänden hatten jedoch gezeigt, dass die Adhäsion an hydrophobe Oberflächen bei pH-Werten oberhalb von 3 zwar deutlich zunahm, bei pH-Werten kleiner 3 jedoch auf fast 0% abnahm (Ref.: Goldberg, S., Doyle, R. J. und Rosenberg, M., 1990, J. Bacteriol. Vol. 172, 5650-5654).

Die Verwendung eines Magnetpartikel-gestützten Verfahrens bietet darüber hinaus noch weitere Vorteile, da ein separates Ernten einzelner Vertiefungen z.B. einer Deepwell-Platte möglich wird und nicht die ganze Platte komplett geerntet werden muß. Somit kann z.B. im Rahmen von Expressionsstudien zeitabhängig geerntet werden.

Zusammenfassend kann festgestellt werden, dass ein einfaches Verfahren zur effizienten, schnellen, kostengünstigen Anreicherung von Bakterienzellen, das ohne einen Zentrifugationsschritt auskommt, und das gleichzeitig für ein breites Spektrum von Bakterien verwendet werden kann, nicht zur Verfügung steht. Daher besteht die Aufgabe der Erfindung in der Bereitstellung eines Verfahrens zur unspezifischen Anreicherung von Bakterienzellen, das ohne Zentrifugationsschritt auskommt.

Die Aufgabe wird durch den in den Patentansprüchen definierten Gegenstand gelöst.

Die Erfindung wird durch die nachfolgenden Figuren näher erläutert:

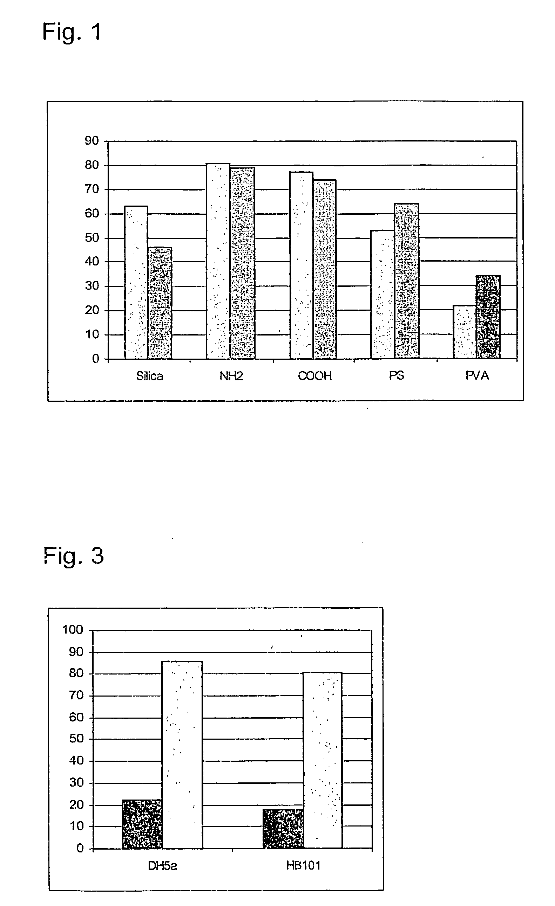

Figur 1 zeigt graphisch das Ergebnis der Zellernteausbeute von E.coli LE392 (helle Balken) und TG2 (dunkle Balken) bei Verwendung verschiedener Magnetpartikel. Die Werte geben die Ausbeute in % an, bestimmt aus dem Verhältnis der Trübung des Überstands und der optischen Dichte der eingesetzten Zellsuspension. Die Abkürzungen bedeuten: Silica: MagPrep Silica Beads (Fa. MERCK, Darmstadt), NH2: Amino-Polystyrol-Beads (Fa. Estapor, MERCK-Eurolab); COOH: Carboxy-Polystyrol-Beads (Fa. Estapor, MERCK-Eurolab); PS: Polystyrol-Beads (Spherotech, Illinois, USA); FVA: Polyvinylalkohol-Beads (Chemagen, Baesweiler)

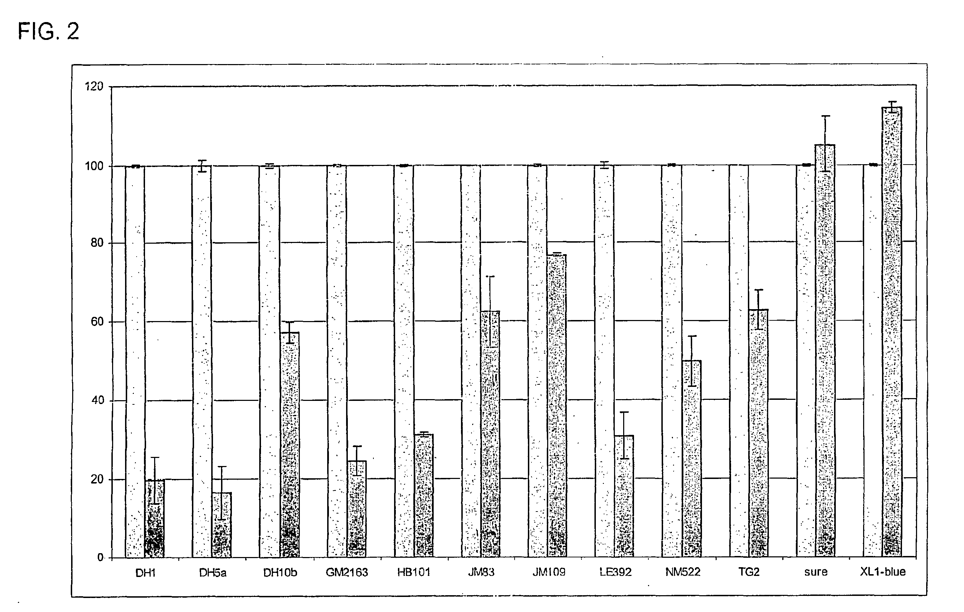

Figur 2 zeigt das Ergebnis der Ausbeute der Zellernte bei saurem pH-Wert mit Silica-Beads (dunkler Balken) und Chitosan/Amino-Polystyrolbeads (heller Balken). In Figur 2 wird die Zellernte-Ausbeute des erfindungsgemäßen Verfahrens (heller Balken) mit dem Verfahren aus WO00/29562 (dunkler Balken) verglichen. Die Zellausbeuten des erfindungsgemäßen Verfahrens sind dabei auf 100% gesetzt, die Ausbeuten nach WO00/29562 wurden relativ dazu bestimmt. Alle Werte sind Mittelwerte aus mehreren Experimenten mit Standardabweichungen. Die Zellernte nach dem erfindungsgemäßen Verfahren wurde wie im Anwendungsbeispiel 5 durchgeführt. Für WO00/29562/MERCK wurde die Vorschrift des Herstellers befolgt.
Figur 3 stellt graphisch das Ergebnis der Zellernte-Ausbeute für die E.coli Stämme DH5a und HB101 bei Verwendung von frei in Lösung befindlichem Chitosan (helle Balken) und an Beads immobilisiertem Chitosan (Chitosan-Beads, Fa. Chemicell, Berlin) (dunkle Balken) dar. Die Werte geben die Ausbeute in % an, ermittelt aus dem Verhältnis der optischen Dichte des Überstands und der eingesetzten Zellsuspension.

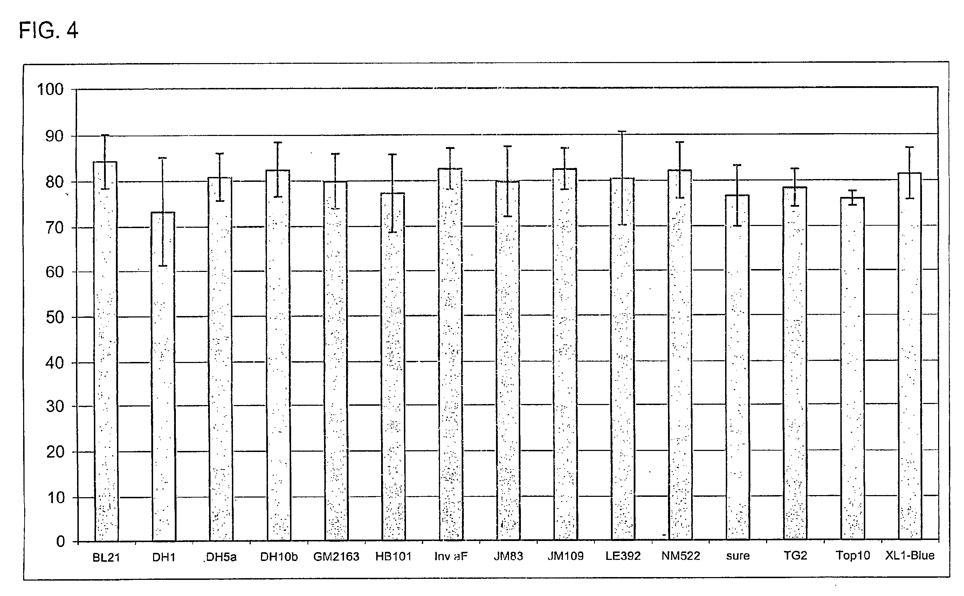

Figur 4 zeigt in einer graphischen Darstellung das Ergebnis der Ausbeute-Effizienz der Zellernte (durchgeführt wie in Anwendungsbeispiel 5) anhand einer Auswahl von 15 E.coli Klonierungsstämnen. Aufgetragen ist die Ausbeute in % (Mittelwerte aus mehreren Experimenten, n=27), ermittelt aus dem Verhältnis der optischen Dichte des Überstands und der eingesetzten Zellsuspension.

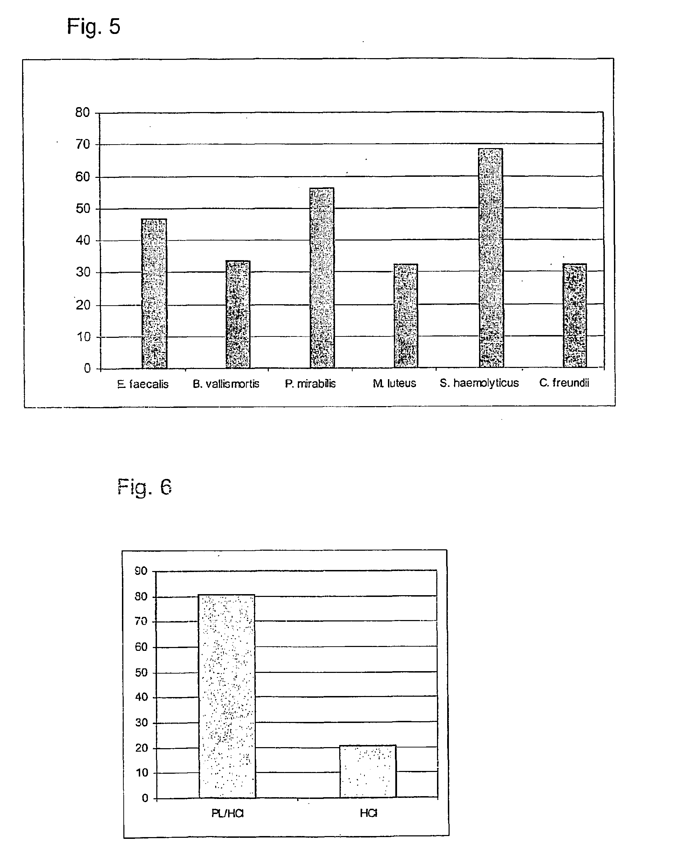

Figur 5 zeigt das Ergebnis der Ernte verschiedener Bakterienarten. Figur 5 zeigt, dass das erfindungsgemäße Zellerntesystem für ein breites Spektrum von Bakterien mehrerer Gruppen (sowohl gram-negativer als auch gram-positiver) angewandt werden kann. Aufgetragen ist jeweils die Ausbeute der Zeliernte. Die Zellen wurden wie in Anwendungsbeispiel 7 geerntet. E. faecalis: Enterococcus faecalis, B. vallismortis: Bacillus vallismortis, P. mirabilis : Proteus mirabilis, M. luteus : Micrococcus luteus, S. haemolyticus : Staphylococcus haemolyticus, C. freundii: Citrobacter freundii.
Figur 6 zeigt graphisch das Ergebnis der Zellernte-Ausbeute für den E.coli Stamm JM83 bei Verwendung von Polylysin in 0.5M HCl bzw. 0.5M HCl ohne Polylysin. Die Werte geben die Ausbeute in % an, ermittelt aus dem Verhältnis der optischen Dichte des Überstands und der eingesetzten Zellsuspension. PL/HCl: Polylysin in 0.5M HCl; HCl: nur 0.5M HCl (Kontrolle ohne Polylysin)

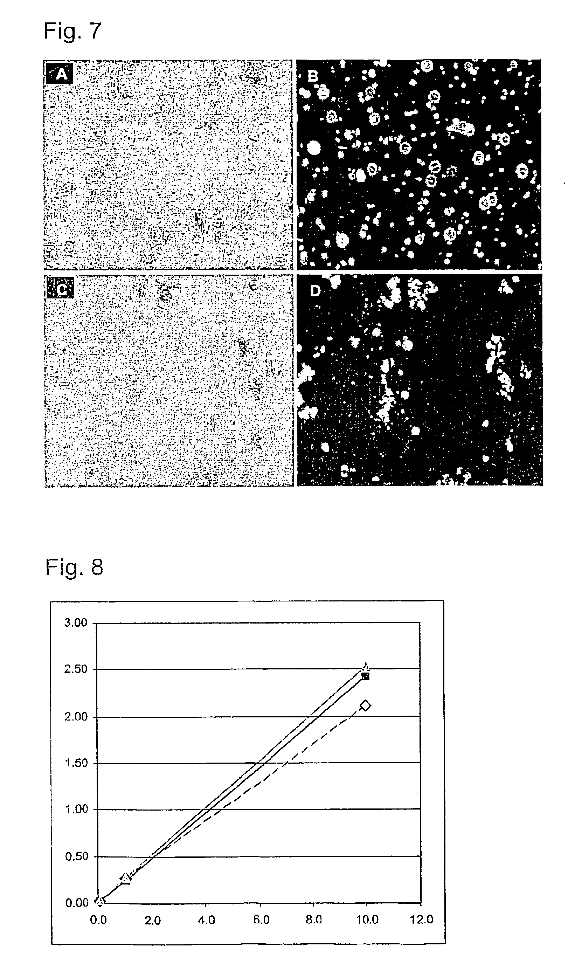

Figur 7 zeigt die Netzwerk-Bildung aus E.coli Zellen, Chitosan und magnetischen Beads. Die Ausbildung dieses Netzes wird durch Zugabe von freiem Chitosan, gelöst in 0.5M Ameisensäure, zu einer E.coli Suspension (Stamm HB101) hervorgerufen (C und D). Im Kontrollansatz ohne Chitosan (nur 0.5M Ameisensäure) entsteht kein Netzwerk (A und B), die Bakterien liegen nach wie vor als Einzelzellen vor. A und C: Hellfeldaufnahmen, in A sind Einzelzellen und Magnetpigmente, in C Aggregate zu sehen. B und D: Fluoreszenzaufnahmen nach Färbung der E.coli Zellen mit Propidiumiodid/Syto9 (BacLightTM Viability Kit, Molecular Probes, Eugene, Oregon,USA). In B sind fluoreszierende Einzelzellen zu sehen, D zeigt die Fluoreszenz der in die Netzstrukturen eingebetteten Zellen.
Figur 8 zeigt graphisch die Enzymaktivität der E.coli-eigenen ß-Galaktosidase nach Zellernte gemäß der vorliegenden Erfindung bzw. nach Ernte über Zentrifugation. Aufgetragen sind die Werte für die Enzymaktivität in relativen Einheiten gegen den in den Test eingesetzten Anteil der aus 300µl geernteten Zellen (in %). Offene Rauten-Symbole geben die Werte für zentrifugierte Zellen wieder. Dreiecke und Quadrate zeigen die Werte für Bakterien, die über das erfindungsgemäße Verfahren geerntet wurden. Dreiecke: die Zellen wurden nach Zugabe von 30µl 125µg/ml Chitosan in 0.5M Ameisensäure für 3 Minuten inkubiert und für weitere 3 Minuten durch Magnetseparation abgetrennt. Quadrate: Die Bakterien wurden nach Zugabe der Lösung für 5 Minuten inkubiert und für weitere 5 Minuten im Magnetfeld separiert.

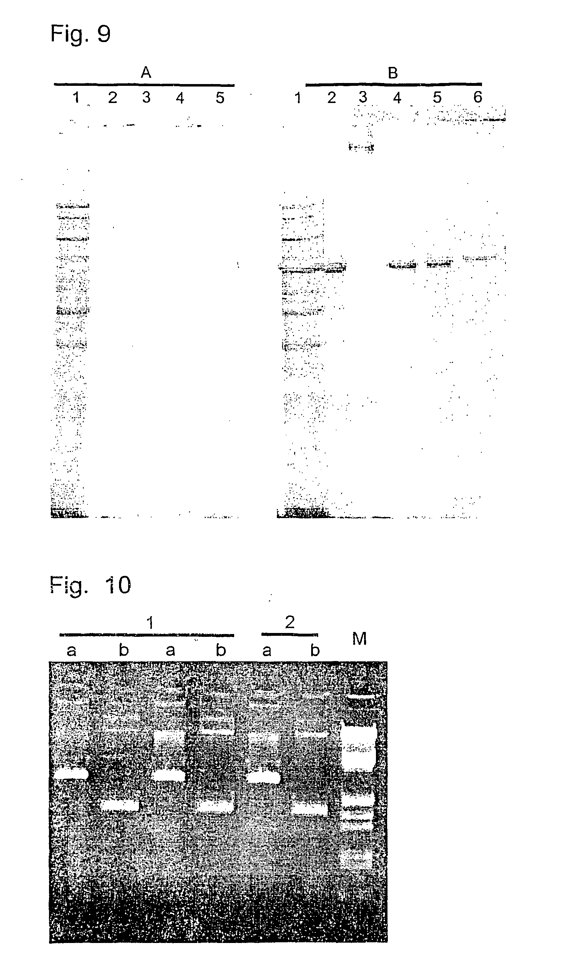

Figur 9 zeigt das Ergebnis der Anreicherung des N-Strep-T4-p12 Proteins über Strep-Tactin Bcads (IBA GmbH, Göttingen) nach Ernte des heterolog exprimierenden E.coli Stammes BL21(DE3) durch das erfindungsgemäße Verfahren. A: Nicht induzierte Zellen. B: Induzierte Zellen; 1: Rohlysat, 2: Strep-Tactin-Beads nach Bindung des N-Strep-T4-p12; 3: eluiertes N-Strep-T4-p12 Protein in nativer, trimerer Form; 4: eluiertes N-Strep-T4-p12 Protein in monomerer Form (nach Erhitzen bei 95°C); 5: Strep-Tactin-Beads nach Elution; 6: Standard (gereinigtes N-Strep-T4-p12 Protein)
Figur 10 zeigt das Ergebnis der Plasmid-DNA Isolierung aus E.coli XL1Blue, geerntet nach dem erfindungsgemässen Verfahren (wie unter Anwendungsbeispiel 4 angegeben). 1: Ernte der Bakterien mittels erfindungsgemässem Verfahren; 2: Ernte der Bakterien durch Zentrifugation; a: Plasmid pUC19 verdaut mit PstI; b: Plasmid unverdaut; M: DNA-Standard (lambda-DNA EcoRI/HindIII).

Der hier verwendete Ausdruck "Aufreinigung" oder "Anreicherung" bedeutet das Trennen von Bakterienzellen oder Zellbestandteilen von der wässrigen Lösung, z.B. dem Kulturmedium, in der sich die Bakterienzellen oder Zellbestandteile befinden. Das Aufreinigen oder Anreichern wird dabei mit Hilfe von Magnetpartikeln durchgeführt.

Der hier verwendete Ausdruck "unspezifische Anreicherung" bedeutet, dass die Anreicherung unter den gewählten Bedingungen unabhängig von Art, Stamm, Gattung etc. der Bakterien durchgeführt wird.

Beschrieben ist ein Verfahren zur unspezifischen Aufreinigung von Bakterienzellen, umfassend die folgenden Schritte:
a) In Kontakt bringen von einer Bakterienzellen enthaltenden Probe mit kationischen Polymeren bei einem sauren pH-Wert oder anionischen Polymeren bei einem basischen pH-Wert,
b) Zugabe eines magnetischen Trägers,
c) Abtrennen des magnetischen Trägers mit den daran gebundenen Bakterienzellen von der Probe.

Ein Aspekt der vorliegenden Erfindung besteht in der Bereitstellung eines Verfahrens zur unspezifischen Aufreinigung von Bakterienzellen, umfassend die folgenden Schritte:
a) In Kontakt bringen von einer Bakterienzellen enthaltenden Probe mit kationischen Polymeren bei einem sauren pH-Wert, wobei sich ein Netzwerk aus Bakterien und Polymeren ausbildet,
b) Zugabe von magnetischen Partikeln, wobei sich die magnetischen Partikel in das Netzwerk aus Bakterien und Polymeren einlagern,
c) Abtrennen der Bakterien über die in dem Netzwerk eingelagerten Magnetpartikel von der Probe.

Im Einzelnen ist das Verfahren durch die folgenden Schritte näher gekennzeichnet:

Die Bakterienkultur wird mit kationischen oder anionischen Polymeren versetzt, insbesondere mit unverzweigten kationischen Polymeren, vorzugsweise mit Chitosan (z.B. FLUKA- Art. Nr. 22741, 22742 oder 22743, Sigma C-3646 oder Roth 5375.1) oder Polylysin (z.B. Sigma P2636). Bevorzugte anionische Polymere sind Dextransulfat, Chondroitinsulfat, Polyglutamat, Polymalat, Polygalacturonsäure, Polyphosphate und sulfatierte Oligosaccharide.

Die Bakterienkultur/Polymer-Mischung muß einen entsprechend den eingesetzten Polymeren in Verbindung mit den entsprechenden Puffern bestimmten pH-Wert haben. Der pH-Wert ist bei kationischen Polymeren vorzugsweise kleiner oder gleich 3 oder 4, bei anionischen Polymeren vorzugsweise größer oder gleich 8. Der pH-Wert kann entweder vor Zugabe der Polymere durch Einstellen der Bakterienkultur mittels Zugabe von Pufferlösungen oder direkt von Säure oder Base auf den gewünschten pH-Wert gebracht werden. Der pH-Wert kann ferner dadurch eingestellt werden, dass die Polymere in einer entsprechenden Pufferlösung oder direkt in Säure oder Base gelöst sind. Der pH-Wert kann ferner dadurch eingestellt werden, dass nach Zugabe der Polymere durch Einstellen der Bakterienkultur/Polymer-Mischung mittels Zugabe von Pufferlösungen oder direkt von Säure oder Base auf den gewünschten pH-Wert gebracht werden.

Die kationischen Polymere können in saurem Puffer oder direkt in Säure gelöst sein. Die Lösung ist so eingestellt, das die Mischung aus Bakterienkultur und kationischen Polymeren den für das Verfahren gewünschten pH-Wert ergibt. Für das erfindungsgemäße Verfahren ist ein saurer pH-Wert der Bakterienkultur/Polymer-Mischung bevorzugt. Der pH-Wert kann mit beliebigen Puffern oder Säuren eingestellt werden, bevorzugt mit 0,5 M HCl/25% Essigsäure, 0,5 M HCl oder 0,5M Ameisensäure. Welcher Puffer verwendet wird, hängt insbesondere von der Art der Weiterverarbeitung der Bakterien bzw. dem anschließenden Verfahren ab. Soll das erfindungsgemäße Verfahren zur Plasmidaufreinigung verwendet werden, ist als Puffer HCl/Hac bei einem pH-Wert im Bereich von etwa 2 bis etwa 3 bevorzugt, da bei einem pH-Wert kleiner als 1,8 die DNA hydrolysiert wird. Soll das erfindungsgemäße Verfahren zur Aufreinigung von Proteinen verwendet werden, ist als Puffer Ameisensäure bei einem pH-Wert im Bereich von etwa 4 bevorzugt, da für die Isolierung von funktionsfähigen Proteinen der pH-Wert im Bereich von etwa 4 bis etwa 9 liegen sollte.

Die kationischen Polymere liegen in einem Konzentrationsbereich vor, in dem eine ausreichende Aufreinigung der Bakterien gewährleistet ist. Polylysin wird für die Fällung mit einer Konzentration im Bereich von 12,5µg/ml Kultur bis 150µg/ml Kultur eingesetzt, vorzugsweise mit einer Konzentration im Bereich von 25µg/ml bis 150µg/ml, insbesondere mit einer Konzentration im Bereich von 40µg/ml bis 75µg/ml, besonders bevorzugt mit einer Konzentration von 50µg/ml. Die optimale Konzentration hängt dabei neben der Bakterienart bzw. dem Bakterienstamm ferner auch von der Bezugsquelle oder dem Molekulargewicht des verwendeten Polylysins ab.

Chitosan wird für die Fällung mit einer Konzentration im Bereich von 0,05µg/ml Kultur bis 100µg/ml Kultur eingesetzt, vorzugsweise mit einer Konzentration im Bereich von 0,25µg/ml bis 50µg/ml, insbesondere mit einer Konzentration im Bereich von 0,5µg/ml bis 50µg/ml, ferner insbesondere mit einer Konzentration im Bereich von 1µg/ml bis 50µg/ml, besonders bevorzugt mit einer Konzentration von 2,5-50 µg/ml. Weitere bevorzugte Konzentrationen, die abhängig vom verwendeten Puffer sein können, ergeben sich aus der nachfolgenden Tabelle. Die optimale Konzentration hängt dabei neben der Bakterienart bzw. dem Bakterienstamm ferner auch von der Bezugsquelle oder dem Molekulargewicht des verwendeten Chitosans und dem Deacetylierungsgrad des Chitosans ab. Eine Übersicht der bevorzugten Chitosan-Konzentrationen findet sich in der nachstehenden Tabelle.

| Chitosan Konzentrationen | Chitosan LMW (FLUKA Art. 22741) | Chitosan MMW Nr. (FLUKA, Art. 22742) | Chitosan HMW Nr. (FLUKA, Art. Nr. 22743 |
|---|---|---|---|
| HCl/HAc | | | |
| Konz.bereich für die Fällung | 0.05µg/ml bis 1 | 0µg/ml 0.05µg/ml bis 5µg/ml | 1µg/ml bis 20µg/ml |
| Optimaler Bereich | 0.5µg/ml bis 5µg/ml | 0.5µg/ml bis 5µg/ml | 1µg/ml bis 5µg/ml |
| Optimum | 2.5µg/ml | | 2.5µg/ml |
| Ameisensäure | | | |
| Konz.bereich für die Fällung | 0.5µg/ml bis 50µg/ml | 0.5µg/ml bis 50µg/ml | 1µg/ml bis 100µg/ml |
| | | | |
| Optimaler Bereich | 0.5µg/ml bis 50µg/ml | 0.5µg/ml bis 50µg/ml | 1µg/ml bis 50µg/ml |
| | | | |
| Optimum | 12.5µg/ml bis 50µg/ml | 12.5µg/ml bis 50µg/ml | 12.5µg/ml bis 50µg/ml |

Nach Zugabe der Polymere sollte die Bakterienkultur/Polymer-Lösung vorzugsweise durch Auf und Abpipettieren oder Schütteln gemischt werden. Durch das Mischen werden alle beteiligten Komponenten schneller und unmittelbar miteinander in Kontakt gebracht.

Die angesäuerte oder basische Polymer/Bakterienmischung kann sofort weiterverarbeitet werden. Die Mischung kann jedoch auch für einen kurzen Zeitraum, vorzugsweise 3-10 Minuten, insbesondere bevorzugt für 5 Minuten inkubiert werden. Die Inkubationstemperatur kann Raumtemperatur bis 40°C, bevorzugt werden 37°C, insbesondere bevorzugt Raumtemperatur betragen.

Die angesäuerte oder basische Polymer/Bakterienmischung wird mit einem magnetischen Träger versetzt. Vorzugsweise sind die magnetischen Träger Magnetpartikel (im weiteren auch Magnetbead genannt), z.B. Polystyrolbeads, oder magnetische Pigmente. Im Rahmen des erfindungsgemäßen Verfahrens können oberflächenfunktionalisierte Polystyrolbeads, insbesondere mit NH2-, COOH- oder OH-Oberflächen, wobei die NH2-Oberfläche bevorzugt ist, eingesetzt werden. Als funktionalisierte Partikel werden bevorzugt käuflich erhältliche Beads z.B. Amino-Beads (EM2 100/40, Durchmesser 1,43µm) oder Carboxy-Beads (EM1 100/40, (23710) Durchmesser 1,3 µm) der Firma Estapor eingesetzt. Als magnetische Pigmente können käuflich erhältliche Beads wie z.B. Bayferrox 318M, Bayoxid E 8706 oder Bayoxid E8713H (Bayer AG) eingesetzt werden. Der bevorzugte Durchmesser der Beads liegt im Bereich von 0,5 µm bis 2 µm, bevorzugt ist ein Durchmesser im Bereich von 1 µm bis 5 µm, besonders bevorzugt von etwa 1 µm.

Das erfindungsgemäße Verfahren beinhaltet die Bildung eines Netzwerkes aus Bakterien und Polymeren in das sich Magnetpartikel egal welcher Art, z.B. Polystyrolbeads oder Pigmente einlagern. Damit können die Bakterien über die in dem Netz befindlichen Magnetpartikel durch Anlegen eines Magnetfeldes abgetrennt werden.

Das erfindungsgemäße Verfahren ist dabei nicht beschränkt auf einen bestimmten Oberflächenfunktionalisierten Magnetpartikeltyp, sondern es kann jede Art von Magnetpartikel, d.h. z.B. Bead oder Pigment, der verschiedensten Oberfläche verwendet werden. Eine Übersicht über gebräuchliche Magnetarten mit verschiedenen Oberflächen ist in Tabelle A und B zusammengestellt. Werden in dem erfindungsgemäßen Verfahren Magnetpigmente verwendet, so können diese sowohl in Alkoholen, als auch in wässrigen Puffern gelöst werden.

Tabelle A stellt tabellarisch alle bisher getesteten und im erfindungsgemäßen Verfahren funktionierenden Magnetpartikel dar. TabelleA: Magnetpigmente, geordnet nach Herstellern. Tabelle B: Magnetische Beads unterschiedlicher Grundmatrizes mit verschiedenen funktionalisierten Gruppen, geordnet nach Herstellern. PS: Polystyrol; Silica: Silica-Matrix; PVA: Polyvinylalkohol

| ***A*** | |
|---|---|
| BASF | Carbonyleisenpulver HQ |
| | Pigment 345 |
| Bayer | Bayferrox 318M |
| | Bayoxide E8706 |
| | Bayoxide E8713 H |
| | Bayoxide E8710 H |
| | Bayoxide E8707 H |
| | Bayoxide E8706 H |
| EPCOS | Ferrite powder N27 |
| MERCK | Iriodin 600 |
| OMIKRON | Eisenhammerschlag Pulver,schwarz |
| | Eisenoxidschwarz farbstark |
| Vogt | Ferrit-Pulver Fi324 |

| ***B*** | | |
|---|---|---|
| estapor | NH₂ | PS |
| | COOH | PS |
| | hydrophob | PS |
| | OH | PS |
| Spherotech | NH₂ | PS |
| | COOH | PS |
| | hydrophob | PS |
| Polysciences | BioMagNH₂ | PS |
| | BioMagCOOH | PS |
| | PS, paramagnetisch | PS |
| | NH₂, paramagnetisch | PS |
| MERCK | MagPrep Silica | Silica |
| Micromod | Sicastar M-NH₂ | Silica |
| | Sicastar M-plain | Silica |
| | Nanomag C, plain | Ferrofluide |
| Chemagen | M-PVA 011, OH | PVA |
| | M-PVA 012, OH | PVA |
| | M-PVA 013, OH | PVA |

Die Magnetpartikel liegen in einem Konzentrationsbereich vor, in dem eine ausreichende Aufreinigung der Bakterien gewährleistet ist, die Kosten der Aufreinigung jedoch in einem vertretbaren Rahmen liegen. Vorzugsweise werden Polystyrolbeads mit einer 1% Polystyrolbead-Lösung in einer Konzentration von etwa 1/10 bis 1/70 des Bakterienkulturvolumens zugesetzt. Besonders bevorzugt ist eine Konzentration von etwa 1/20 bis 1/50, insbesondere von 1/20 oder 1/50. Die Konzentration hängt dabei von der Größe des Volumens der Bakterienkultur ab. Beträgt das Volumen der Bakterienkultur z.B. 0,2 oder 1 oder 1,5 ml, so ist eine Konzentration der 1% Polystyrolbead-Lösung von 1/20 bevorzugt, bei einem Volumen der Bakterienkultur von z.B. 10 ml ist eine Konzentration der 1% Polystyrolbead-Lösung von 1/50 bevorzugt. Je größer das Volumen der Bakterienkultur, so geringer kann die Konzentration der Polystyrolbeads sein. Die magnetischen Pigment werden vorzugsweise als 5%-Lösung mit einer Konzentration von 1/10 Volumen der Bakterienkultur eingesetzt.

Nach Zugabe der Magnetpartikel sollte die Bakterienkultur/Polymer/Magnetpartikel-Lösung vorzugsweise durch Auf- und Abpipettieren, Schütteln oder mittels des Magneten gemischt werden. Durch das Mischen werden alle beteiligten Komponenten schneller und unmittelbar miteinander in Kontakt gebracht.

Die Mischung kann sofort weiterverarbeitet werden. Die Mischung kann jedoch auch für einen kurzen Zeitraum, vorzugsweise 3-10 Minuten, insbesondere bevorzugt für 5 Minuten inkubiert werden. Die Inkubationstemperatur kann Raumtemperatur bis 40°C, bevorzugt werden 37°C, insbesondere bevorzugt Raumtemperatur betragen.

Anschließend werden die an die magnetischen Träger gebundenen Bakterien von der Probe abgetrennt. Dabei wird das Gemisch aus Bakterien/Polymer/Magnetpartikeln in ein magnetisches Feld eingebracht und der Komplex abgeschieden.

Die durch den vorhergehenden Verfahrensschritt an die Bakterien gebundenen Magnetpartikel können je nach Art des folgenden Weiterverarbeitungschrittes - z.B. Plasmidpräparation, Präparation von RNA oder genomischer DNA - von den Bakterien z.B. durch Schütteln abgetrennt und verworfen oder in den Weiterverarbeitungschritten mit den Bakterien zusammen weiterverwendet werden. Sollen die Bakterien von den Magnetpartikeln getrennt werden, können die Bakterien/Polymer/Magnetpartikel nach Abtrennen von der Probe erneut in entsprechende Puffer aufgenommen und bei gleichzeitigem Zurückhalten der Magnetpartikel mittels des Magneten abgenommen und z.B. in eine neues Reaktionsgefäß überführt werden. Zum Lösen der Bakterien aus dem Bakterien/Polymer/Magnetpartikel-Netzwerk wird vorzugsweise ein Tris-Puffer (200mM, pH 8 - 9,5) verwendet. Zum Lösen wird vorzugsweise ein hochtouriger Schüttelschritt bei z.B. 1200 rpm (Eppendorf Schüttler) durchgeführt.

Das erfindungsgemäße Verfahren kann in allen üblichen Reaktionsgefäßen durchgeführt werden, vorzugsweise in Eppendorf-Reaktionsgefäßen aller Art oder Mikrotiterplatten.

Die mit dem erfindungsgemäßen Verfahren angereicherten Bakterien können alle Arten von Bakterien, vorzugsweise Eubacteria und Archaebakteria, besonders E. coli, insbesondere BL21, JM109, E.coli B, DH1, LE392, E.coli K12, DH5α, NM522, E.coli C, DH10b, Sure, GM2163, TG2, HB101, Top10, InvaF', XL1Blue, JM83, aber auch Micrococcus spec. insbesondere Micrococcus luteus, Proteus spec. insbesondere Proteus mirabilis, Enterococcus spec. insbesondere Enterococcus faecalis, Citrobacter spec. insbesondere Citrobacter freundii, Bacillus spec. insbesondere Bacillus vallismortis, Staphylococcus spec. insbesondere Staphylococcus haemolyticus sein.

Das erfindungsgemäße Verfahren kann als Zentrifugationsersatz verwendet werden, und somit die automatische Aufreinigung von Bakterienzellen ermöglichen. Mit dem beschriebenen Verfahren können Bakterien aus einer Kultur innerhalb von etwa 5-10 Minuten unspezifisch angereichert werden. Da die Konzentration der verwendeten kationischen Polymere mit deutlich unter 1% sehr gering ist, können die Anreicherungen wesentlich kostengünstiger als bei Verfahren nach dem Stand der Technik durchgeführt werden. Ferner werden beim erfindungsgemäßen Verfahren wesentlich geringere Bead-Mengen von 1-15 Beads pro Bakterium statt 30-50 Beads pro Bakterium z.B. bei der WO00/29562 eingesetzt.

Die folgenden Beispiele dienen nur der Erläuterung und beschränken in keiner Weise den Umfang der Erfindung.

### 1. Herstellung der Chitosanlösung

Chitosan wurde eingewogen, in 0.5N HCl/25%HAc aufgenommen und die Lösung wurde solange erhitzt (ca. 80-90°C) bis sich das Chitosan vollständig gelöst hatte. Anschliessend wurde die Lösung verdünnt und auf 25µg/ml in 0.5N HCl/25%HAc (=working solution H) oder auf 125µg/ml in 0.5N Ameisensäure (=working solution A) eingestellt. Die working solution (25µg/ml) bleibt bei Raumtemperatur stabil in Lösung und wurde so gelagert.

### 2. Herstellung der Polylysinlösung

Polylysin wurde eingewogen, in 0.5N HCl aufgenommen, auf 0.5mg/ml in 0.5N HCl (=working solution) eingestellt. Die working solution wurde bei -20°C gelagert bis zum Gebrauch.

### 3. Bakterienernte im Maßstab 0,2ml Kultur: Netzbildung

Die Anzucht von E. coli HB101 erfolgte über Nacht in einer 0.5ml 96Well Polypropylen-Mikrotiterplatte mit 0.2 ml Kultur pro Well. Zur Kultur wurden 20µl Chitosan working-solution A, bzw. 20µl 0.5 M Ameisensäure ohne Chitosan im Kontrollansatz und 20µl einer 5%igen Lösung von Magnetpigment Bayoxid E8706 in PBS gegeben und durch Auf- und Abpipettieren gemischt. Nach einer Inkubation bei Raumtemperatur für 5 Minuten wurden die Proben mikroskopiert, um die Netzbildung zu überprüfen (Figur 7).

### 4. Bakterienernte im Maßstab 1,5 ml Kultur: DNA-Isolierung

Die Anzucht von E.coli XL1Blue transformiert mit Plasmid pUC19 erfolgte in einer 96er DWP über Nacht mit je 1.25ml Kulturvolumen pro Well in LB-Medium mit 100µg/ml Ampicillin. Zur Übemachtkultur wurden dann 125µl Chitosan-working solution H und 125µl einer 5%igen Pigmentbeads-Lösung (Bayferrox 318M in Isopropanol) gegeben und durch Auf- und Abpipettieren gut gemischt. Nach einer Inkubation bei Raumtemperatur für 5 Minuten wurden die Bakterien/Beads-Komplexe auf einem Magnetseparator (Bilatec AG, Mannheim) abgetrennt. Der Überstand wurde vollständig abgenommen und die Plasmid-DNA wurde aus dem Zell-Sediment über den DNA-Isolierungs-Kit Wizard MagneSil™ Plasmid Purification System (Promega, Madison, USA) nach Vorschrift des Herstellers isoliert (Fig. 10)

### 5. Bakterienernte im Maßstab 10 ml Kultur

Die Anzucht von E.coli JM83 erfolgte in einem Polypropylenröhrchen über Nacht mit 10ml Kulturvolumen. Nach der Übemacht-Kultur wurde 1 ml (1/10 Volumen) Chitosan-working solution H zugegeben und durch zweimaliges Auf- und Abpipettieren gemischt. Danach wurden 0,2 ml einer 1%igen Amino-Polystyrolbead-Lösung zugegeben. Anschliessend wurde die Lösung durch Auf- und Abpipettieren (2 fach) gemischt. Es folgte eine Inkubation bei Raumtemperatur für 5 Minuten. Das Röhrchen wurde 10 Minuten in das Magnetfeld eines Permanentmagneten (Bilatec AG, Mannheim) zur Sedimentation der Bakterien/Bead-Komplexe gebracht. Der Überstand wurde verworfen und die Bakterienzellen direkt weiterverarbeitet.

### 6. Anzucht der E.coli Kulturen

E.coli Kulturen wurden in Standardmedien wie LB, 2xYT, SOB oder SOC (Maniatis, 1987) über Nacht angezogen. Zu je 200µl der Übemachtkulturen wurden je 20µl Chitosan working-solution H und je 20µl Amino-Polystyrol-Beads gegeben und gut durchmischt. Nach 5 Minuten Inkubation erfolgte die Abtrennung der Bakterien/Beads-Komplexe auf einem Magnetseparator. Bei Verwendung der genannten Medien kann gegebenenfalls eine Anpassung der Konzentrationen von working-solution und Beads nötig sein.

### 7. Bakterienernte im Maßstab 0,2 ml Kultur

Die Anzucht von je 0,2 ml Kulturen von Enterococcus faecalis, Proteus mirabilis, Staphylococcus haemolyticus erfolgte in einer 96er Mikrowellplatte über Nacht bei 37°C. Die Anzucht von je 0,2 ml Kulturen von Bacillus vallismortis, Micrococcus luteus, Citrobacter freundii erfolgte in einer 96er Mikrowellplatte über Nacht bei 30°C. Zu der Übemacht-Kultur wurde 20 µl (1/10 Volumen) Chitosan-working solution H zugegeben. Danach wurden 10 µl einer 1%igen Amino-Polystyrolbead-Lösung zugegeben. Anschliessend wurde die Lösung im Well durch Auf- und Abpippettieren (1 oder 2 fach) gemischt. Es folgte eine Inkubation bei Raumtemperatur für 5 Minuten. Die Mikrotiterplatte wurde 5 Minuten auf einem Magnetseparator (Bilatec AG, Mannheim) zur Sedimentation der Bakterien/Bead-Komplexe inkubiert. Der Überstand wurde verworfen und die Bakterienzellen direkt weiterverarbeitet.

### 8. Ernte lebender Bakterien

Zu je 300µl einer ÜN Kultur von E.coli BL21(DE3) wurden in einer 0.5ml 96well PP-Mikrotiterplatte je 30µl Chitosan-working solution A und 30µl einer 5%igen Pigmentbeads-Lösung (Bayferrox 318M in PBS) zugegeben und durch Auf- und Abpipettieren gut gemischt. Danach folgte ein Inkubationsschritt bei Raumtemperatur für 3 bzw. 5 Minuten. Anschliessend wurden die Bakterien/Beads-Komplexe durch Inkubation für 3 bzw. 5 Minute auf einem geeigneten Magnetseparator (Bilatee AG, Mannheim) sedimentiert. Der Überstand wurde verworfen und die Bakterien/Beads-Pellets in je 200µl 200mM Tris pH 8.0 resuspendiert. Als Kontrolle wurden 300µl der ÜN-Kultur abzentrifugiert und das Pellet ebenfalls in 200µl 200mM Tris pH 8.0 resuspendiert. Die Lebensfähigkeit der Zellen wurde durch Ausplattieren von Verdünnungsreihen auf Agarplatten überprüft und Kolonien nach ÜN-Inkubation ausgezählt. Als weiterer Test für die Aktivität und Lebensfähigkeit der Zellen wurde die Enzymaktivität der zelleigenen ß-Galaktosidase (Apte et al., 1995, Wat. Res. 29, 1803-1806) gemessen (Fig. 8).

### 9. Proteinisolierung:

Zur Proteinisolierung aus mit dem erfindungsgemäßen Verfahren geernteten Bakterien wurde das Strep-Tag System eingesetzt. Dazu wurde p12 (Selivanov, N.A., et al., 1988 Nucleotide and deduced amino acid sequence of bacteriophage T4 gene 12, Nucleic Acids Res.;16(5): 2334) mit nach molekularbiologischen Standardverfahren mit einem N-terminalen Strep-Tag (US 5506121) fusioniert und wie von Burda, M.R. & Miller S. (Europ. J. Biochem, 1999, 265: 771 - 778) beschrieben exprimiert. Die Anzucht des N-Strep-T4-p12 exprimierenden E.coli Stammes BL21(DE3) pNS-T4-p12p57 erfolgte in einer 96er Deepwellplatte mit 1.25ml Kultur pro well. Bei einer Zelldichte OD600 von ca. 0.5 wurde mit 1mM IPTG für 3h induziert. Anschliessend wurden pro well 125µl Chitosan-working solution A und 125µl einer 5%igen Pigmentbeads-Lösung (Bayoxid E8706 in PBS) zugegeben, durch Auf- und Abpipettieren gut durchmischt und für 5 Minuten bei Raumtemperatur inkubiert. Danach wurden die Bakterien/Beads-Komplexe durch 5- minütige Inkubation auf einem geeigneten Magnetseparator (Bilatec AG, Mannheim) sedimentiert. Der Überstand wurde vollständig entfernt und das Pellet in 150µl Puffer A (200mM Tris pH 8.0, 200mM NaCl, 10mM EDTA, 0.05% Tween20) resuspendiert. Die Bakterien wurden anschliessend durch einen Schüttelschritt (5 Minuten bei 1200rpm, Schüttler der Fa. Eppendorf) aus dem Bakterien/Beads-Netz gelöst. Durch nochmalige Inkubation auf dem Magnetseparator wurden die Beads von den nun im Überstand befindlichen Zellen abgetrennt und der Überstand in ein neues Well einer 0.5ml 96well PP-Mikrotiterplatte überführt. Die Zellen wurden anschliessend durch Zugabe von Lysozym (1,6 mg/ml), 2 Minuten schütteln bei 750rpm und 15 Minuten Inkubation bei Raumtemperatur lysiert und die DNA durch Zugabe von 5µg/ml DNAse verdaut. Das heterolog exprimierte N-Strep-T4-p12 wurde durch Zugabe von 10µl 5%iger StrepTactin-Beads (Firma IBA, Göttingen) und 20 Minuten Inkubation unter leichtem Schütteln an die StrepTactin-Beads gebunden und aus dem Lysat angereichert. Durch Zugabe von 5mM Biotin wurde das N-Strep-T4-p12 von den Beads eluiert (Fig. 9).

## Patentansprüche

1. Verfahren zur unspezifischen Aufreinigung von Bakterien, umfassend die folgenden Schritte:
a) In Kontakt bringen von einer Bakterienzellen enthaltenden Probe mit einem kationischen Polymer bei einem sauren pH-Wert, wobei sich ein Netzwerk aus Bakterien und Polymeren ausbildet
b) Zugabe von magnetischen Partikeln, wobei sich die magnetischen Partikel in das Netzwerk aus Bakterien und Polymeren einlagern
c) Abtrennen der Bakterien über die in dem Netzwerk eingelagerten Magnetpartikel von der Probe.

2. Verfahren nach Anspruch 1, wobei nach Schritt a) und/oder Schritt b) ein Inkubationsschritt eingefügt wird.

3. Verfahren nach einem der vorhergehenden Ansprüche, wobei das kationische Polymer Chitosan oder Polylysin ist.

4. Verfahren nach einem der vorhergehenden Ansprüche, wobei Chitosan mit einer Konzentration im Bereich von 0,05 bis 100 µg/ml Kultur und Polylysin mit einer Konzentration im Bereich von 12,5 bis 150 µg/ml Kultur zugegeben wird.

5. Verfahren nach einem der vorhergehenden Ansprüche, wobei der pH-Wert mit HCl/Hac auf kleiner oder gleich 3 oder mit Ameisensäure auf kleiner oder gleich 4 eingestellt wird.

6. Verfahren nach einem der vorhergehenden Ansprüche, wobei die magnetischen Partikel einen Durchmesser von 0,5 bis 5 µm haben:

7. Verfahren nach einem der vorhergehenden Ansprüche, wobei die magnetischen Partikel ein magnetisches Pigment oder ein magnetisches Polystyrolbead, insbesondere mit einer NH2-, COOH- oder OH-Oberfläche sind.

8. Verfahren nach einem der vorhergehenden Ansprüche, wobei die aufgereinigten Bakterien E.coli, Bacillus spec, Proteus spec, Micrococcus spec, Staphylococcus spec oder Citrobacter spec sind.

## Claims

1. A method for the unspecific purification of bacteria comprising the following steps:
a) contacting the sample, containing bacterial cells, with a cationic polymer at an acidic pH- value, wherein a network is formed from bacteria and polymers
b) addition of a magnetic particles, wherein the magnetic particles incorporate into the network of bacteria and polymers
c) separation of the bacteria via magnetic particles incorporated into the network from the sample.

2. The method according to claim 1, wherein after step a) and/or step b) an incubation step is inserted.

3. The method according to any of the preceding claims, wherein the cationic polymer is chitosan or polylysine.

4. The method according to any of the preceding claims, wherein chitosan is added with a concentration in the range from 0.05 to 100 µg/ml culture and polylysine with a concentration in the range of 12.5 to 150 µg/ml culture.

5. The method according to any of the preceding claims, wherein the pH-value is adjusted with HCl/HAc to less than or equal to 3 or with formic acid to less than or equal to 4.

6. The method according to any of the preceding claims, wherein the magnetic particles have a diameter of 0.5 to 5 µm.

7. The method according to any of the preceding claims, wherein the magnetic particles are a magnetic pigment or a magnetic polystyrene-bead, in particular with a NH₂-, COOH- or OH-surface.

8. The method according to any of the preceding claims, wherein the purified bacteria are E.
*coli, Bacillus spec, Proteus spec, Micrococcus spec, Staphylococcus spec* or *Citrobacter spec.*

## Revendications

1. Procédé de purification non spécifique de bactéries, comprenant les étapes suivantes :
a) mise en contact d'un échantillon contenant des cellules bactériennes avec un polymère cationique à un pH acide, un réseau de bactéries et de polymères se formant,
b) addition de particules magnétiques, les particules magnétiques s'incrustant dans le réseau de bactéries et de polymères,
c) séparation des bactéries sur les particules magnétiques incrustées dans le réseau et de l'échantillon.

2. Procédé selon la revendication 1, dans lequel une étape d'incubation est insérée après l'étape a) et/ou l'étape b).

3. Procédé selon l'une des revendications précédentes, dans lequel le polymère cationique est le chitosane ou la polylysine.

4. Procédé selon l'une des revendications précédentes, dans lequel le chitosane est ajouté à une concentration dans la plage allant de 0,05 à 100 µg/ml de culture et la polylysine est ajoutée à une concentration dans la plage allant de 12,5 à 150 µg/ml de culture

5. Procédé selon l'une des revendications précédentes, dans lequel le pH est ajusté avec du HCl/Hac à une valeur inférieure ou égale à 3 ou avec de l'acide formique à une valeur inférieure ou égale à 4.

6. Procédé selon l'une des revendications précédentes, dans lequel les particules magnétiques ont un diamètre allant de 0,5 à 5 µm.

7. Procédé selon l'une des revendications précédentes, dans lequel les particules magnétiques sont un pigment magnétique ou une bille de polystyrène magnétique, en particulier avec une surface NH₂, COOH ou OH.

8. Procédé selon l'une des revendications précédentes, dans lequel les bactéries purifiées sont *E. coli, Bacillus spec., Proteus spec., Micrococcus spec., Staphylococcus spec.* ou *Citrobacter spec.*
